# EUROPÄISCHE PATENTSCHRIFT

(11) **EP 1 766 385 B1**
(45) Veröffentlichungstag und Bekanntmachung des Hinweises auf die Patenterteilung: **15.03.2017**
(21) Anmeldenummer: 05759036.6
(22) Anmeldetag: 08.07.2005
(51) Int. Cl.: G01N 33/00

(54) **MESSEINRICHTUNG ZUM MESSEN MINDESTENS EINES UMWELTPARAMETERS**
MEASURING DEVICE FOR MEASURING AT LEAST ONE ENVIRONMENTAL PARAMETER
DISPOSITIF DE MESURE POUR MESURER AU MOINS UN PARAMETRE DE L'ENVIRONNEMENT

(30) Priorität: 12.07.2004 AT 48804 U; 15.12.2004 AT 91104 U
(43) Veröffentlichungstag der Anmeldung: 28.03.2007
(73) Patentinhaber: mlu-recordum Environmental Monitoring Solutions GmbH, 2351 Wiener Neudorf (AT)
(72) Erfinder: KILGUS, Traugott, A-2340 Mödling (AT)
(74) Vertreter: Schwarz & Partner
(86) Internationale Anmeldenummer: PCT/AT2005/000260
(87) Internationale Veröffentlichungsnummer: WO 2006/005093

(56) Entgegenhaltungen:
- US-A- 4 818 348
- US-A- 5 801 297
- US-A- 5 832 411
- US-A1- 2002 178 789
- US-B1- 6 495 341

## Beschreibung

Die Erfindung betrifft eine Messvorrichtung gemäß Anspruch 1 zum Messen mindestens eines Umweltparameters mit mindestens einem Sensor, wobei vom Sensor erfasste Umweltparameter in Messwerte physikalischer Größen umgerechnet werden und über eine Datenfernverbindung abrufbar sind, sowie eine Messeinrichtung hierzu.

### Hintergrund der Erfindung

Die Verunreinigung der Umwelt mit Schadstoffen hat Auswirkungen auf die Lebensqualität und gegebenenfalls auch die Gesundheit der sich in dieser Umwelt aufhaltenden Personen, aber auch Konsequenzen hinsichtlich der Erhaltbarkeit der entsprechenden Fauna und Flora. Diese Erkenntnis führt einerseits zur Notwendigkeit, Verfahren zur Beurteilung der Umwelt-Qualitätsparameter für die relevanten Schadstoffe zu entwickeln und diese je nach Einschätzung des Risikos regelmäßig bzw. laufend anzuwenden, um einerseits die Einhaltung von Grenzwerten, die durch Gesetze oder Richtlinien vorgegeben sind, zu überwachen und andererseits Potentiale für Maßnahmen zu erkennen, welche das Erreichen eines Zielwertes ermöglichen. Dabei ist zu berücksichtigen, dass die Schadstoffkonzentrationen räumlich und zeitlich variieren.

Vor diesem Hintergrund wurden in den letzten 30 Jahren weltweit Umweltqualitäts-Überwachungsnetze aufgebaut, welche unter Nutzung der verfügbaren Technologie die erforderlichen Messungen durchführen. Die dabei einzusetzenden Verfahren und Beurteilungsmethoden sind in vielen Anwendungsbereichen detailliert genormt, was zwar einerseits der Vergleichbarkeit der Beurteilungsergebnisse nützt, aber andererseits die Durchsetzung neuer Verfahren und innovativer Technologie hemmt. Im Bereich der Luftgüte-Messung sind beispielsweise die in den Instrumenten eingesetzten Messverfahren im wesentlichen seit den frühen 70er Jahren unverändert.

Die Erkenntnisse aus den in diesem Zeitraum gewonnenen Daten im Vergleich mit medizinischen Daten, also die epidemologische Forschung hinsichtlich der Auswirkung von Luftschadstoffen, führt zur Festlegung von Grenzwerten und Zielwerten für die einzelnen Schadstoffe. Dabei ist zwischen den im Jahresmittel nicht zu überschreitenden Grenzwerten und solchen, die auch bei kurzfristiger Überschreitung zu Maßnahmen führen müssen, den Alarmschwellen, zu unterscheiden. Durch die technologische Weiterentwicklung auf der Seite der Emittenten (beispielsweise durch Einsatz von Katalysatortechnologie oder niedriger verschwefelter Kraftstoffe), aber auch durch Verschiebung der Zusammensetzung der Grundgesamtheit aller Emittenten (beispielsweise steigender Fahrzeuganteil mit Dieselantriebsaggregaten oder höherer Anteil von Biomasse-Festfeuerungen) können sich die Schwerpunkte der erforderlichen Beurteilungen über die Zeit verschieben, wie dies der Rückgang der Bedeutung von Schwefeldioxid-Monitoring einerseits, aber der signifikante Anstieg der Bedeutung der Beurteilung der Feinstaub (PM10) Konzentration andererseits gut darstellen. Die entsprechenden Überwachungssysteme müssen daher eine entsprechende Flexibilität hinsichtlich der zu erfassenden Schadstoff-Vielfalt aufweisen.

In den letzten Jahren konzentriert sich die Aufmerksamkeit der für die Einhaltung der Luftqualität zuständigen Stellen immer mehr auf die sogenannten "Hot Spots", also Gebiete, in denen auf Grund von besonderen Bedingungen, wie hohe Konzentration an Emittenten, besondere meteorologische Lagen, oder ähnliches, in erhöhtem Maß die Grenzwerte und auch fallweise die Alarmschwellen überschritten werden. Solche Gebiete können entweder als permanente Hot Spots ausgeprägt sein, beispielsweise an besonders frequentierten Verkehrsknoten, oder aber auch zeitlich begrenzt, beispielsweise während der Durchführung von Großbauvorhaben, auftreten.

Der Stand der Technik ist durch eine dichotome Situation geprägt:
Einerseits kommen hoch zeitauflösend messende automatisierte Messstationen, die eine Vielzahl von Schadstoffen erfassen, zum Einsatz. Diese bestehen üblicherweise aus containerartigen, klimatisierten Gebäuden oder Aufbauten, welche die in ihrem Inneren montierten Laborgeräte (typisch pro Schadstoff je ein Analyseinstrument mit Netzversorgung, Sensor, Signalaufbereitung, interner Messwertberechnung, Anzeige und Bedienteil sowie Schnittstellen zur Kommunikation mit einem Leitrechner) vor Wetter und Zugriff durch nicht qualifizierte Personen schützen. Entsprechende normgerechte Probenahmesysteme sowie Instrumente zur zeitsynchronisierten Erfassung von meteorologischen Daten (Regen, Temperatur, Windstärke und -richtung) ergänzen den Messaufbau. Typisch enthält eine solche Station noch eine lokale Datenerfassungseinheit (Stationsrechner, Logger, oder Ähnliches), welche dann mittels Datenfernübertragung die Messwerte an die Messnetz-Zentrale weiterleitet, wo die Analyse mittels spezialisierter Software durchgeführt wird. Wie aus der vorstehenden Beschreibung nachvollziehbar, handelt es sich bei diesen Stationen um aufwendige Installationen, die auf Grund Ihrer Abmessungen und der für den Betrieb erforderlichen Versorgungssysteme sowie der damit verbundenen Investitionen typisch Gebiete von mehreren bis mehreren hundert Quadratkilometern je Station abdecken, und sich damit nicht für den - allenfalls temporären - Einsatz an einer Vielzahl von Hot Spots eignen.

Andererseits gibt es die nach dem Diffusionsgesetz nach Fick funktionierenden Passiv- oder Diffusionssammler, welche hinsichtlich der erfassten Anzahl an unterschiedlichen Schadstoffen gemäß der Anzahl und Type der pro Sammelstelle montierten Diffusionssammler begrenzt ist, wobei ein einzelner Diffusionssammler typisch nur für einen Schadstoff geeignet ist, in besonderen Fällen aber auch bis zu drei Schadstoffe gleichzeitig erfasst werden können. Auf Grund der geringen Größe der einzelnen Sammler, es handelt sich dabei typisch um Röhrchen mit max. einigen 100 mm Länge und einem Durchmesser von typisch 10-20 mm, entspricht daher eine Messanordnung mit 4 solchen Diffusionssammlern in seiner Größe den typischen Brutkästen für Singvögel und ist also hinsichtlich der Kompaktheit der Abmessungen für regional hoch aufgelöste Messungen geeignet. Die typische Mittelungszeit, also jene Zeitspanne, für die ein einzelner Messwert ermittelt werden kann, beträgt jedoch bei allen Diffusionssammlern 7 bis 14 Tage. Da diese auch noch zur Analyse in ein Labor verbracht und dort mittels Desorption und weiterer Analyseverfahren ausgewertet werden müssen, können Beurteilungen über die Schadstoffkonzentration im Messgebiet nicht in Echtzeit zur Verfügung gestellt werden. Eine Erfassung der örtlichen meteorologischen Bedingungen während der Messung ist ebenfalls nicht gegeben.

In der konventionellen Technik kommen autarke Analysatoren zum Einsatz. Dies hat sowohl historische als auch praktische Gründe. Die historischen Gründe liegen in der laufenden Entwicklung von Analysatoren für die jeweils relevanten Schadstoffe.

Jeder neue Schadstoff bekam einen neuen Analysator, und dieser wurde wiederum in-einem Messrack hinzugefügt, um in der Messstation einen neuen Schadstoff messen zu können.

Solche Analysatoren messen in der Regel nur einen Schadstoff pro Analysator. Auch dies hat historischen Hintergrund, welcher im wesentlichen in der Leistungsfähigkeit der Elektronik begründet ist. Mit den notwendigen regeltechnischen und messtechnischen elektronischen Bauelementen sowie dem Netzteil, der Pumpe und dem eigentlichen Messsensor, der Anzeige und der Bedienung war ein typisches 19" Messgerät vollständig angefüllt. Trotzdem waren die Geräte in hohem Maße empfindlich gegen Temperatur- und Luftfeuchteschwankungen, wie dies für Laborgeräte typisch ist. Daher wurden die Geräte in klimatisierte Messräume integriert.

Auf dieser Architektur bauen auch heute noch die Normen für Luftgütesysteme auf.

Im Regelfall besteht daher jeder Analysator für gasförmige Luftschadstoffe aus messgeräteinternen Probeleitungen, welche das Prüfgut (Messgas) von einer zentralen Probenahme, die wiederum in Form, Größe und Ausführung in Normen beschrieben ist, übernehmen und es zuerst zu einem Partikelfilter führen, welcher durch Herausfiltern von Staub die zumeist optischen Messsysteme vor Verschmutzung schützt. Danach führt die Leitung zu einem Sensor. Die eigentlichen Messprinzipien des Sensors sind physikalischer Natur und seit Jahrzehnten unverändert. Sie sind als sogenannte Referenzmethoden ebenfalls in der Norm festgeschrieben.

In einer Messstation, die auf Grund der notwendigen manuellen Eingriffe so gestaltet sein muss, dass diese den Sicherheitsvorschriften gemäß durchgeführt werden können und dass die Analysatoren vor unbefugtem Fremdzugriff geschützt sind, sind zumeist in zumindest einem Messrack alle Analysatoren in einer Zusammenbauform, wie sie in der industriellen Prozesstechnik üblich ist, einer über dem anderen montiert. Alle Geräte werden über einen in der Messstation eingebauten Schaltschrank versorgt.

Die Analysatoren sind für die Aufstellung in Innenräumen konzipiert und reagieren auf Schwankungen der Umgebungsbedingungen (Lufttemperatur und -feuchtigkeit) zumindest mit Messwertschwankungen, bei extremeren Abweichungen auch mit Geräteausfall.

In den bekannten Messstationen muss daher zur Einhaltung der Messqualität die Raumtemperatur und Luftfeuchte innerhalb bestimmter Schwankungsbreiten gehalten werden. Dazu wird einerseits ein sich aus der Dimension der Station ergebendes Luftvolumen genutzt, welches als thermische Masse dient und diese mittels einer geeignet dimensionierten konventionellen Klimaanlage hinsichtlich der Eckdaten Lufttemperatur und -feuchte innerhalb der für die gewünschte Messgenauigkeit erforderlichen Bandbreite hält.

Die für den ordnungsgemäßen Betrieb des Messsystems erforderliche Leistung ist dabei bekanntermaßen von der Größe des umbauten Volumens, den Wärmedurchgangswerten der Begrenzungswände, den Außenbedingungen sowie der Zahl und Leistung der Abwärme produzierenden Verbraucher abhängig. Letztere bestehen einerseits aus der Abwärme von Hilfseinheiten, wie Netztransformatoren, Pumpen und ähnliches, und andererseits aus den sich aus den eingesetzten Messverfahren ergebenden besonderen Abwärmequellen, wie beispielsweise Infrarot-Strahler oder beheizte Katalysatoren.

Für übliche dem Stand der Technik entsprechende Messstationen beträgt der Leistungsbedarf auf Grund der Baugrößen und der hohen Zahl an Abwärmequellen typisch mehrere kW, sodass zumeist auch Leistungsstromanschlüsse für den Betrieb vorhanden sein müssen.

Eine zentrale Probenahmeeinheit mit Hauptpumpe sorgt für entsprechenden Durchfluss an Messgut, aus dem die Analysatoren wiederum ihr Messgas entnehmen.

Die Datensammlung erfolgt in der Regel durch einen Datenlogger mit integriertem Microprozessor, welcher die Daten von den Messgeräten als Master anfordert und entsprechend zusammen mit einem Zeitwert speichert. Dabei stehen lediglich bereits fertig berechnete Messwerte der einzelnen Analysatoren zur Speicherung zur Verfügung. In manchen Ausführungen lässt sich daneben eine Kalibriertabelle mit Zero und Span Informationen ablegen, die eine Bewertung der Messergebnisse zulässt. Die meisten Datenlogger verfügen heute über einen lokalen Massenspeicher, wie beispielsweise eine Festplatte, auf der die abgefragten Messwerttabellen zwischengespeichert werden können.

Über ein Modem werden diese Tabellen dann entweder nach vorprogrammiertem (zeitabhängigem) Muster über eine Datenfernübertragung an einen Leitrechner in der Messzentrale geschickt. In der Regel wird dort die statistische Bewertung z.B. in Tagesmittelwerte oder ähnliches vorgenommen und von dort aus der Öffentlichkeit zur Verfügung gestellt. Diese Zur-Verfügung-Stellung für die Öffentlichkeit kann auch mittels Übertragung an einen Webserver erfolgen.
US 5,801,297 offenbart eine transportable Messeinrichtung mit einer Vielzahl von gekapselten Sensoren-Clustern, die jeweils in einem Gehäuse angeordnet sind.
US2002/0178789 offenbart eine transportable Messeinrichtung, bei der ein Chip mit einer Vielzahl von Sensoren modular in die Einrichtung eingschoben werden kann. Die Chips können separat gekühlt bzw. geheizt werden.

Die Erfindung bezweckt die Vermeidung der oben geschilderten Schwierigkeiten und Nachteile der Messeinrichtungen gemäß dem Stand der Technik und stellt sich die Aufgabe, eine Messeinrichtung hochkompakt und allenfalls portabel zu gestalten, wobei Schwankungen der Umgebungsbedingungen keine bzw. nur unwesentliche Messwertschwankungen verursachen können. Insbesondere soll eine erfindungsgemäße Messeinrichtung auch als Datenauswertestation fungieren können, und zudem soll eine Transparenz zwischen dem Rohsignal, d.h. dem Spannungssignal, und den daraus resultierenden physikalischen Messwerten gegeben sein. Neben der Minimierung des erforderlichen Raumbedarfs, z.B. Grundfläche, ist dabei auch die Reduktion der Versorgungsanforderungen, beispielsweise Strombedarf, ein wesentliches Ziel der Erfindung.

Diese Aufgabe wird bei einer Messeinrichtung zum Messen mindestens eines Umweltparameters nach dem erfindungsgemäßen Verfahren, mit einem geschlossenen Gehäuse, zentraler Stromversorgung und wenigstens einer Probenahme und zugehöriger Pumpe und wenigstens einem Sensor ist zur Lösung der der Erfindung zu Grunde liegenden Aufgabe gekennzeichnet dadurch, dass der Innenraum des Gehäuses hinsichtlich der thermischen Regelung in einen lediglich fremdbelüfteten Versorgungsraum und einen mit geregelten Messbedingungen versehenen Messraum aufgeteilt ist und Abwärme abstrahlende Sensoren bzw. deren Subkomponenten, wie beispielsweise Katalysatoren, durch eine thermische Isolierung einzeln gegenüber dem Messraum geschirmt sind.

Vorteilhafte Weiterbildungen einer solchen Messeinrichtung sind in den Unteransprüchen 2 bis 25 enthalten.

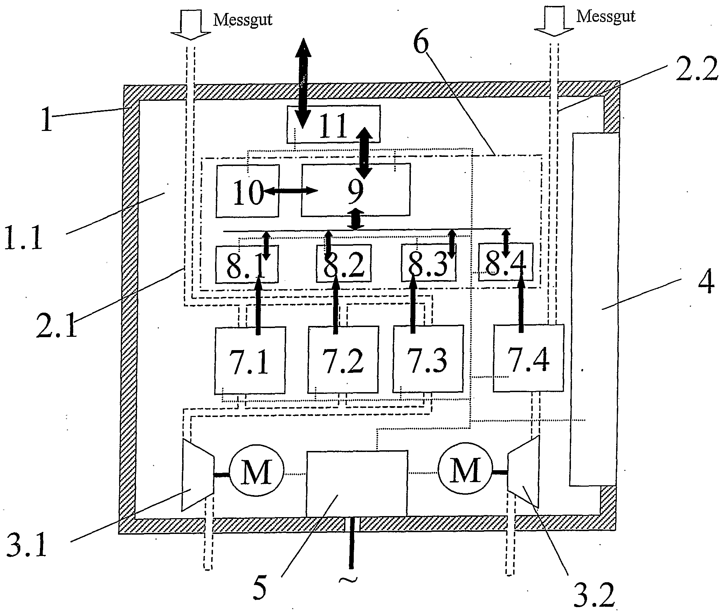

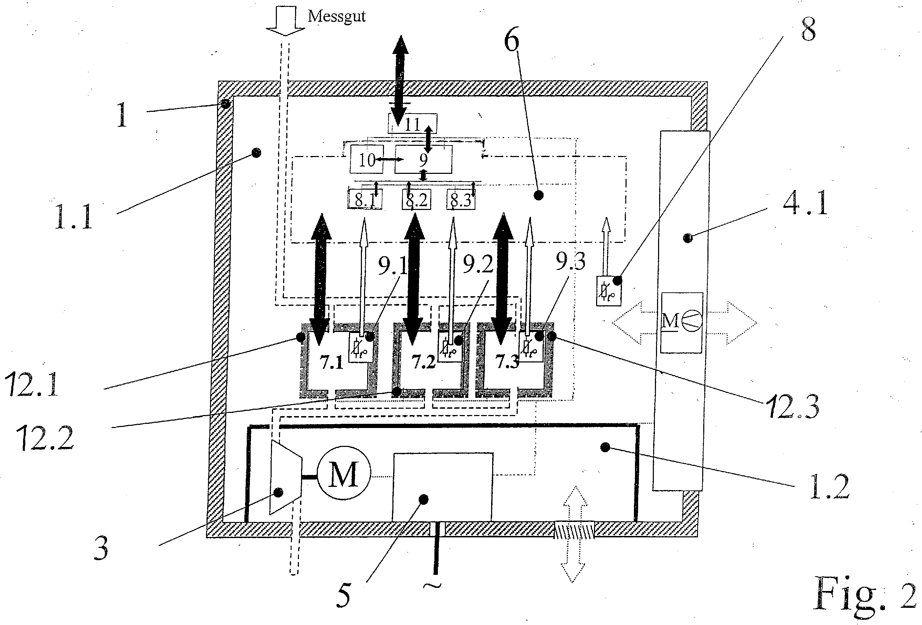

Die Erfindung ist nachfolgend anhand zweier in der Zeichnung schematisch in Blockschaubilddarstellung wiedergegebener Ausführungsbeispiele näher erläutert, wobei Fig. 1 keine erfindungsgemäße Ausführungsform darstellt und Fig. 2 ein Ausführungsbeispiel gemäß der Erfindung ist.

Gemäß Fig.1 ist eine kompakte Messeinrichtung zur Messung von Umweltparametern aus folgenden Elementen gebildet: ein kompaktes, wetterfestes und thermisch isolierendes Gehäuse 1 aus Metall oder Kunststoff mit zwei Probenahmen 2.1, 2.2 und den zugehörigen Pumpen 3.1, 3.2, sowie einer integrierten Vorrichtung 4 zur Klimatisierung des Gehäuseinnenraums 1.1 und der zentralen Stromversorgung 5. Darin eingebaut ist als Kern-Funktionselement die Datenerfassungs- und -verarbeitungseinheit 6 mit modularem Aufbau, wobei in der ersten Verarbeitungsebene eine Architektur mit verteilten Prozessen eingesetzt wird, wobei die Messsignale jedes im System eingesetzten Sensors 7.1 ... 7.n zu einem eigenen Signal-Prozessor 8.1 ... 8.n geführt werden. Dort erfolgt die Umsetzung in Digitalwerte sowie allfällige für die Applikation erforderliche oder sinnvolle statistische Bewertung, beispielsweise Mittelung. Am Ausgang des Signal-Prozessors steht ein allfällig bewerteter, digitalisierter Spannungswert des Sensors an, welcher in dem Zentral-Prozessor 9 in mit physikalischen Größen versehene Messwerte, beispielsweise Konzentrationen, umgerechnet wird und in der Folge auf einem lokalen Massenspeicher 10 gespeichert werden kann oder über die notwendigen Schnittstellen zu unterschiedlichen Ausführungsformen für Datenfernübertragung 11 Daten zur Verfügung stellt. Da der Zentral-Prozessor 9 auch Webserver-Funktionalität besitzt, erfolgt die Datenbereitstellung für den Benutzer mittels Browser-Software, so dass ohne Spezialsoftware von jedem Computer mit Internet-Browser und -anschluss die Messdaten abgefragt werden können.

Die zweite Probenahme wird nur gebraucht, wenn man Feinstaub messen will. In diesem Fall ist die Probenahme ein wesentlicher Teil des Messverfahrens, da sie als scharfkantige Abscheider ausgeprägt ist, und damit nur jene Partikel abgeschieden werden, die einer bestimmten Durchmesserklassifizierung entsprechen. Alle gasförmigen Schadstoffe werden mit einer einzigen Probenahme gemessen.

Das System ist je nach Messaufgabe unterschiedlich konfigurier- und erweiterbar. Die Konfiguration erfolgt durch Hinzufügen bzw. Ersatz von Sensoren und der zugeordneten ersten Signalverarbeitungsebene. Typische Konfigurationen für die beispielhaften Anwendungen sind:
Einrichtungen zur klassischen Immissions- oder Emissionsmessung umfassen ein bis fünf Sensoren, wobei hier unterschiedliche Messverfahren (Beispielsweise Non Dispersive Infrarot Sensoren für Kohlenmonoxid bzw. -dioxid, Chemiluminiszenz für Stickoxide, UV-Photometrie für Ozon, UV-Flourseszenz für Schwefeldioxid, Schwefelwasserstoff und ähnliche Schwefelverbindungen, aber auch Non-Dispersive UV-Absorption für Stickoxide sowie PID Sensoren für flüchtige organische Substanzen oder FID Sensoren für die Bestimmung von Kohlenwasserstoffkonzentrationen) zum Einsatz kommen. Zusätzlich wird typisch ein Sensor für Feinstaub bzw. Partikelkonzentrationsmessung integriert, wobei für diesen eine weitere Probenahme erforderlich ist.

Besondere Anwendungen sind Einrichtungen zum qualitativen Monitoring, welche beispielsweise Arrays von Halbleiter-Sensoren, z.B. aus Metalloxid, einsetzen.

In einer weiteren Ausführung können die Sensoren zur Erfassung der relevanten meteorologischen Daten, wie beispielsweise Windgeschwindigkeit und -richtung, oder Luftdruck und -temperatur sowie Luftfeuchte durch An- bzw. Einbau von Sensoren in das System integriert werden.

In einer weiteren Ausführung, beispielsweise für Anwendungen im Verkehrsbereich, kann ein Sensor zur Erfassung von Lärmparametern, wie Schalldruck, in das System integriert werden.

In einer besonders vorteilhaften Ausführung für die Messung von Luftqualität wird die Erfindung in eine allenfalls hinterleuchtungsfähige Litfasssäule oder ein Großbilddisplay derart integriert, dass sowohl Werbe- als auch Messfunktion gesichert sind.

In einer weiteren vorteilhaften Ausführung für die Anwendung als Einrichtung zur Überwachung von Schadstoffkonzentrationen in Flüssigkeiten, insbesondere aber nicht ausschließlich Wasser, werden beispielsweise Sensoren zur Bestimmung des Gehaltes an Nitraten, Phosphaten, Total Organic Content (TOC), pH-Wert oder des biologischen Sauerstoffbedarfes (BSB) und ähnlicher Messgrößen eingesetzt. Diese arbeiten vorwiegend nach elektrochemischen Verfahren, setzen ionenselektive Membranen ein oder nutzen Redox-Effekte. Gebräuchlich sind auch photometrische Sensoren oder solche, welche die elektrische Leitfähigkeit der Flüssigkeit als Messverfahren einsetzen. Im Bereich TOC kommen vor allem Messverfahren mit Aufschließung der organischen Substanzen durch Hochtemperaturoxidation oder Beigabe von Reagenzien, wie Borsäure, und Messung der dabei entstehenden CO₂ Konzentration zum Einsatz.

Fig. 2 zeigt eine Ausführungsform der Erfindung mit minimiertem Hilfsenergieaufwand. Die Messeinrichtung gemäß Fig. 2 weist ein thermisch isolierendes Gehäuse 1 aus Metall oder Kunststoff auf, wobei der Gehäuseinnenraum zum verbesserten thermischen Management in mindestens zwei Teilräume, nämlich den Messraum 1.1 mit den Sensoren 7.1...7.n sowie der Datenverarbeitungseinheit 6 und den separat belüfteten Versorgungsraum 1.2 mit wenigstens einer Probenahmepumpe 3 und wenigstens einer Stromversorgung 5 bzw. Netzversorgungseinheit aufgeteilt ist, und die im Messraum 1.1 angeordneten Sensoren 7.1 ... 7.n bzw. jene für das thermische Management relevanten Bauelemente, wie beispielsweise beheizte Katalysatoren zu den Sensoren durch individuell angepasste Isolationselemente 12.1 bis 12.n nochmals thermisch abgeschirmt sind und in der zugehörigen, modular aufgebauten Datenerfassungs- und -verarbeitungseinheit 6 eine elektronische Klimaregelung der Messbedingungen im Messraum geführt nach den Messwerten eines im Messraum angebrachten Temperatursensors 8 und eine Überwachung der Betriebstemperaturen der Messsensoren oder deren separat isolierter Komponenten mittels Temperatursensoren 9.1...9.n erfolgt, wobei bei Überschreitung der zulässigen Betriebstemperaturen der thermisch isolierten Komponenten eine Abschaltung ausgelöst wird.

Je nach Type, Konfiguration bzw. Anzahl der Messsensoren 7.1 ... 7.n erfolgt die Kühlung entweder über eine an einer Gehäuseaußenwand angebrachte konventionelle Kompressionsklimaanlage 4 oder über ein an der gleichen Stelle montiertes Kühlaggregat 4.1 bestehend aus wenigstens einem Peltierelement und einer Zwangsbelüftung.

Die so realisierte Temperaturregelung ermöglicht den Betrieb der kompakten Einrichtung zur Messung von Umweltparametern ohne wesentliche Einbußen in der Messgenauigkeit, jedoch mit elektrischen Anschlussleistungen, welche typisch nur 10 % des typischen Bedarfes konventioneller Messstationen darstellt und jedenfalls unter 0,5 kW bleibt und damit immer mittels einphasiger Stromversorgung realisiert werden kann.

Die Erfindung bietet gegenüber dem Stand der Technik folgende Vorteile:
1. Entfall einer Reihe von redundanten Bauelementen, die allerdings erst beim Vergleich mit einer Station mit mehr als einem Sensor wirklich sichtbar werden: So arbeitet die erfindungsgemäße Messeinrichtung mit nur einem Netzteil für alle Sensoren und benötigt keinerlei Anzeigen und Bedieneinheiten. Mehrere bzw. alle Sensoren für gasförmige Schadstoffe werden mit einer gemeinsamen Pumpe betrieben. Hierdurch kann eine bisher noch nicht erzielte Kompaktheit bzw. Minimierung des Raumbedarfes der Messeinrichtung erzielt werden.
2. Das thermische Management des Systems ist auf minimale Wärmeabstrahlung der einzelnen Elemente ausgelegt und damit der Klimatisierungsbedarf bzw. der dafür erforderliche Energieaufwand deutlich reduziert.
3. Bei einem konventionellen Analysator wird das digitalisierte Analogsignal in der Messeinrichtung komplett verarbeitet und als fertiger Messwert ausgegeben. Das System ist dabei geschlossen, ein Zugriff von außen auf die "internen Werte", welche zu den einzelnen Messwerten führen, ist nicht möglich. Erfindungsgemäß kommt ein verteiltes Prozessorsystem zum Einsatz. Die Analog/Digital Konversion wird in einem integrierten Microcontroller durchgeführt (erste Prozessorebene) und - so vom übergeordneten Systemrechner (zweite Prozessorebene) entsprechend angefordert - auch gleich eine erste statistische Bewertung, beispielsweise Mittelung, der digitalisierten Spannungssignale vorgenommen. Die Statussignale und Soll/Ist-Werte der einzelnen geräteinternen Messbedingungen - die wiederum mit denen konventioneller Analysatoren identisch sind bzw. sein können, da ja aufgrund der Vorschriften der Norm hinsichtlich der Referenzmethoden neben anderen Typen auch die gleichen Sensoren wie in den konventionellen Analysatoren "bedient" werden müssen - werden von der ebenfalls durch einen Microcontroller gesteuerten Sensorregelungsebene (erste Prozessorebene) ebenfalls über den geräteeigenen Bus zum übergeordneten Systemrechner übertragen. Im Gegensatz zu konventionellen Analysatoren ist diese Sensorregelungsebene aber nicht ausschließlich auf einen Sensor definiert, sondern kann eine Vielzahl von Sensoren, darunter auch elektrochemische Sensoren oder Halbleitersensoren, versorgen. Mit den Informationen aus der ersten Prozessorebene werden dann in der zweiten Prozessorebene die Umrechnungen in physikalische Messwerte auf Basis der Kalibrierdaten durchgeführt. Alle Daten stehen gemeinsam in einer Echtzeit-Datenbank. Damit wird eine derzeit einzigartige Transparenz vom Rohsignal des Sensors, den Randbedingungen der Messung (Globalparameter und Statusparameter des Messgeräts) bis zum fertigen Messwert geschaffen. Die Datenbank ist dann, z.B. über einen Webserver, in unterschiedlicher Form zugänglich. Der normale Benutzer kann die Daten in entsprechender, im System hinterlegter Form betrachten, erweiterte User dürfen bestimmte Daten downloaden oder zu einem Server übertragen, wo sie mit anderen Messwerten, wie die einer konventionellen Station, in ein Überwachungssystem integriert werden können. Advanced User oder Service-Personal haben von jedem Rechner der Welt- aber auch von jedem webfähigen Mobiltelefon oder PDA - zu jeder Zeit Zugriff auf die Volldatenbank in Echtzeit und können somit die Funktion des Systems, wie beispielsweise aber nicht ausschliesslich Lampenspannungen oder Lüfterdrehzahlen, überprüfen. Durch Übertragen von Datenfiles können darüber hinaus einzelne Parameter aktiv verändert werden oder sogar komplette Softwareelemente, welche ansonsten in den Geräten typisch Firmware-Charakter haben, ausgetauscht werden. Darüber hinaus kann das System dank der Webserverfunktion auch mittels üblicher webbasierter Funktionen, wie z.B. e-Mail, Alarm oder sonstige Statusmeldungen übertragen, und so allenfalls einen Austausch zu ermöglichen, noch bevor ein Ausfall einer Komponente die Messwerte ungültig macht. Der Zugang zur Außenwelt, insbesondere dem Internet, erfolgt dabei natürlich konventionell, d.h. via Modem, Funk/GSM/GPRS/UMTS oder W-LAN Verbindung, da ansonsten ja keine Anbindung an das Internet möglich ist.
4. Im Unterschied zu allen anderen derzeit in diesem Bereich im Einsatz befindlichen Systemen ist für die Kommunikation mit der erfindungsgemäßen Messeinrichtung keinerlei herstellerspezifische Software - nicht ein Mal ein sogenannter Treiber - erforderlich, lediglich einer der als Standardsoftware installierten Web-Browser (MS Internet Explorer, Netscape Navigator, Mozilla, etc.) muss laufen und man muss die Internetadresse des Gerätes kennen und das für die gewünschten Privilegien erforderliche Passwort besitzen.

Insgesamt führt das zu einem gegenüber der kleinsten bekannten in konventioneller Architektur ausgeführten mehrkomponententauglichen Stationen zu einem Miniaturisierungsfaktor von ca. 10 und gegenüber den sogenannten Vollstationen zu einem Miniaturisierungsfaktor von ca. 100. Der Energieverbrauch fällt von typisch 3500 W und damit 3Phasen Wechselstrom (Drehstrom) auf typisch 350 W für die Messung von drei Schadstoff-Komponenten und damit klar 1 Phasen Wechselstrom mit dem Potential zur mobilen, netzunabhängigen Stromversorgung. Die Messqualität bleibt aber mit der von konventionellen Vollstationen vergleichbar.

## Patentansprüche

1. Messeinrichtung zum Messen mindestens eines Umweltparameters, mit einem geschlossenen Gehäuse (1), in dem eine zentrale Stromversorgung (5), wenigstens eine Probenahme (2) und zugehörige Pumpe (3), wenigstens ein Sensor (7.1, 7.2, ... 7.n) und eine Datenerfassungs- und -verarbeitungseinheit (6) mit modularem Aufbau angeordnet sind, **dadurch gekennzeichnet,**
**dass** der Innenraum des Gehäuses (1) hinsichtlich der thermischen Regelung in einen lediglich fremdbelüfteten Versorgungsraum (1.2) und einen Messraum (1.1) mit einer integrierten Vorrichtung (4, 4.1) zur Klimatisierung des Messraums (1.1) aufgeteilt ist und Abwärme abstrahlende Sensoren (7.1, 7.2, ... 7n) oder deren Subkomponenten, wie beispielsweise Katalysatoren, durch eine thermische Isolierung (12.1, 12.2, ... 12.n ) einzeln gegenüber dem Messraum (1.1) abgeschirmt sind, wobei die Pumpe (3) und die Stromversorgung (5) in dem Versorgungsraum (1.2) angeordnet sind und die Datenerfassungs- und -verarbeitungseinheit (6) und der wenigstens eine Sensor (7.1, 7.2, ...) in dem Messraum (1.1) angeordnet sind.

2. Messeinrichtung nach Anspruch 1, **gekennzeichnet durch** eine als Kompressionsklimaanlage ausgebildete Vorrichtung (4) zur Klimatisierung des Messraums (1.1).

3. Messeinrichtung nach Anspruch 1, **gekennzeichnet durch** eine Vorrichtung (4.1) zur Klimatisierung des Messraums (1.1) mit wenigstens einem zwangsbelüfteten Peltierelement.

4. Messeinrichtung nach einem der Ansprüche 1 bis 3, **gekennzeichnet durch** Temperatursensoren (9.1, 9.2, ... 9.n) zur Überwachung der Temperatur der Sensoren (7.1, 7.2, ...) oder deren Subkomponenten unter den Isolierungen (12.1, 12.2,... 12.n).

5. Messeinrichtung nach einem der Ansprüche 1 bis 4, **dadurch gekennzeichnet, dass** die Datenerfassungs- und -verarbeitungseinheit (6) bei anhaltender Überschreitung der zulässigen Betriebstemperatur der Sensoren (7.1, 7.2, ... 7.n) oder Subkomponenten unter den individuellen Isolierungen ( 12.1, 12.2, ... 12.n ) diese abschaltet.

6. Messeinrichtung nach einem der Ansprüche 1 bis 5, **dadurch gekennzeichnet, dass** die Datenerfassungs- und -verarbeitungseinheit (6) einen modularem Aufbau mit mindestens einem Signal-Prozessor (8.1, ... 8.n) zur Aufnahme und Umsetzung der Messsignale
zumindest eines Sensors (7.1, 7.2, ... 7.n) in Spannungswerte und einem an den mindestens einen Signal-Prozessor (8.1 ... 8.n) gekoppelten Zentral-Prozessor (9) zur Umrechnung der Spannungswerte in Messwerte physikalischer Größen aufweist, wobei der Zentral-Prozessor (9) gekoppelt ist mit einem lokalen Messwertspeicher (10).

7. Messeinrichtung nach Anspruch 6, **dadurch gekennzeichnet, dass** der Zentral-Prozessor (9) mit einer Schnittstelle einer Datenfernübertragungseinheit (11) verbunden ist.

8. Messeinrichtung nach Anspruch 6 oder 7, **dadurch gekennzeichnet, dass** der Zentral-Prozessor (9) eine Webserverfunktion aufweist.

9. Messeinrichtung nach Anspruch 3, **dadurch gekennzeichnet, dass** das Peltier-Element ein an einer Außenwand des Gehäuses (1) montiertes geregeltes Peltier-Element ist.

10. Messeinrichtung nach Anspruch 1, **gekennzeichnet durch** eine als Kleinstklimaanlage ausgebildete Vorrichtung (4) zur Klimatisierung des Messraums (1.1), welche in die Außenwand des Gehäuses (1) integriert ist.

11. Messeinrichtung nach einem der Ansprüche 1 bis 10, **dadurch gekennzeichnet, dass** als Sensoren (7.1) für die Bestimmung von Gaskonzentrationen Sub-Einheiten eingesetzt sind, welche nach einem der physikalischen Messverfahren NDIR, NDUV, PID, FID, Chemiluminiszenz oder UV-Absorption arbeiten.

12. Messeinrichtung nach einem der Ansprüche 1 bis 11, **dadurch gekennzeichnet, dass** als Sensor (7.1) zur Messung von Sauerstoffkonzentration in Gasen Sub-Einheiten eingesetzt sind, welche nach elektrochemischem Prinzip mit Zirkonoxid oder nach paramagnetischem Prinzip arbeiten.

13. Messeinrichtung nach einem der Ansprüche 1 bis 12, **dadurch gekennzeichnet, dass** als Sensoren (7.1) für die Bestimmung von Gaskonzentrationen Sub-Einheiten eingesetzt sind, welche aus wenigstens einem Halbleiter-Sensor aufgebaut sind.

14. Messeinrichtung nach einem der Ansprüche 1 bis 13, **dadurch gekennzeichnet, dass** als Sensoren (7.1) für die Bestimmung von Gaskonzentrationen Sub-Einheiten eingesetzt sind, welche nach dem elektrochemischen Prinzip aufgebaut sind.

15. Messeinrichtung nach einem der Ansprüche 1 bis 14, **dadurch gekennzeichnet, dass** zusätzlich eine Sub-Einheit (7.n) zur Messung von Partikelkonzentration in Gasen mittels Microwaage, Lichtstreuung oder -extinktion-Verfahren vorgesehen ist.

16. Messeinrichtung nach einem der Ansprüche 1 bis 15, **dadurch gekennzeichnet, dass** zusätzlich Sensoren (7.n) für Windgeschwindigkeit, Windrichtung, Lufttemperatur, Luftdruck und Luftfeuchte, welche in die Messeinrichtung integriert sind, vorgesehen sind.

17. Messeinrichtung nach einem der Ansprüche 1 bis 16, **dadurch gekennzeichnet, dass** zusätzlich eine Sub-Einheit (7.n) für die Messungen von Lärmparametern, wie beispielsweise Schalldruck, vorgesehen ist.

18. Messeinrichtung nach einem der Ansprüche 1 bis 17, **dadurch gekennzeichnet, dass** Sensoren (7.n) zur Bestimmung von Schadstoffgehalten in Flüssigkeiten, beispielsweise Wasser, als Subeinheiten vorgesehen sind, welche nach coloumetrischen Verfahren arbeiten.

19. Messeinrichtung nach einem der Ansprüche 1 bis 18, **dadurch gekennzeichnet, dass** Sensoren (7.n) zur Bestimmung von Schadstoffgehalten in Flüssigkeiten, beispielsweise Wasser, als Subeinheiten eingesetzt sind, welche nach elektrochemischen Verfahren, insbesondere auch unter Einsatz von ionenselektiven Membranen, arbeiten.

20. Messeinrichtung nach einem der Ansprüche 1 bis 19, **dadurch gekennzeichnet, dass** Sensoren (7.n) zur Bestimmung von Schadstoffgehalten in Flüssigkeiten, beispielsweise Wasser, als Subeinheiten eingesetzt sind, welche mittels der Bestimmung der elektrischen Leitfähigkeit arbeiten.

21. Messeinrichtung nach einem der Ansprüche 1 bis 20, **dadurch gekennzeichnet, dass** Sensoren (7.n) zur Bestimmung von Schadstoffgehalten in Flüssigkeiten, beispielweise Wasser, als Subeinheiten eingesetzt sind, welche nach photometrischen Verfahren arbeiten.

22. Messeinrichtung nach einem der Ansprüche 1 bis 21, **dadurch gekennzeichnet, dass** Sensoren (7.n) zur Bestimmung von Schadstoffgehalten in Flüssigkeiten, beispielsweise Wasser, als Subeinheiten eingesetzt sind, welche den TOC (Total Organic Compounds) Gehalt über Verfahren der Aufschließung der organischen Substanzen über Umwandlung in Kohlendioxid, beispielsweise durch Hochtemperaturbehandlung oder Einsatz chemischer Reagenzien, wie beispielsweise Borsäure, bestimmen.

23. Messeinrichtung nach einem der Ansprüche 1 bis 22, **dadurch gekennzeichnet, dass** wenigstens ein Sensor (7.n) zur Bestimmung des pH-Wertes von Flüssigkeiten, beispielsweise Wasser, eingesetzt ist.

24. Messeinrichtung nach einem der Ansprüche 1 bis 23, **dadurch gekennzeichnet, dass** wenigstens ein Sensor (7.n) zur Bestimmung des BSB (Biologischen Sauerstoff Bedarfes) von Flüssigkeiten, beispielsweise Wasser, eingesetzt ist.

25. Messeinrichtung nach einem der Ansprüche 1 bis 24, **dadurch gekennzeichnet, dass** sie in ein Werbemedium, wie beispielsweise in eine Litfasssäule oder in ein Großdisplay, integriert ist.

## Claims

1. A measuring device for measuring at least one environmental parameter, comprising a closed casing (1) in which a central current supply (5), at least one sampling (2) and an associated pump (3), at least one sensor (7.1, 7.2,...7.n) and a data acquisition and processing unit (6) having a modular design are arranged, **characterized in that** the interior of the casing (1) is divided, in terms of thermal control, into a merely force-ventilated supply room (1.2) and a measurement room (1.1) comprising an embedded device (4, 4.1) for air-conditioning the measurement room (1.1) and sensors (7.1, 7.2,...7.n) emitting waste heat or their subcomponents, respectively, such as, for example, catalysts are shielded individually from the measurement room (1.1) via thermal insulation (12.1, 12.2, ...12.n), wherein the pump (3) and the current supply (5) are arranged in the supply room (1.2) and the data acquisition and processing unit (6) and the at least one sensor (7.1, 7.2, ...) are arranged in the measurement room (1.1).

2. A measuring device according to claim 1, **characterized by** a device (4) designed as a compression air conditioning system for air-conditioning the measurement room (1.1).

3. A measuring device according to claim 1, **characterized by** a device (4.1) for air-conditioning the measurement room (1.1) comprising at least one force-ventilated Peltier element.

4. A measuring device according to any of claims 1 to 3, **characterized by** temperature sensors (9.1, 9.2,... 9.n) for monitoring the temperature of the sensors (7.1, 7.2, ...) or their subcomponents beneath the insulations (12.1, 12.2, ... 12.n).

5. A measuring device according to any of claims 1 to 4, **characterized in that**, in case the admissible operating temperature of the sensors (7.1, 7.2,... 7.n) or subcomponents, respectively, beneath the individual insulations (12.1, 12.2, ... 12.n) is persistently exceeded, these can be switched off by the data acquisition and processing unit (6).

6. A measuring device according to any of claims 1 to 5, **characterized in that** the data acquisition and processing unit (6) has a modular design comprising at least one signal processor (8.1 ... 8.n) for receiving the measuring signals of at least one sensor (7.1, 7.2,... 7.n) and converting them into voltage values and a central processor (9) coupled to the at least one signal processor (8.1 ... 8.n) for converting the voltage values into measured values of physical variables, the central processor (9) being coupled to a local memory of measured values (10).

7. A measuring device according to claim 6, **characterized in that** the central processor (9) is connected to an interface for a long-distance data transmission unit (11).

8. A measuring device according to claim 6 or 7, **characterized in that** the central processor (9) has a web server function.

9. A measuring device according to claim 3, **characterized in that** the Peltier element is a controlled Peltier element mounted to an outer wall of the casing (1).

10. A measuring device according to claim 1, **characterized by** a device (4) designed as a micro air conditioning system for air-conditioning the measurement room (1.1), which device is embedded in the outer wall of the casing (1).

11. A measuring device according to any of claims 1 to 10, **characterized in that** subunits operating according to any of the physical measuring methods NDIR, NDUV, PID, FID, chemiluminescence or UV absorption are included as sensors (7.1) for the determination of gas concentrations.

12. A measuring device according to any of claims 1 to 11, **characterized in that** subunits operating according to the electrochemical principle with zirconium oxide or according to the paramagnetic principle are included as sensors (7.1) for measuring the oxygen concentration in gases.

13. A measuring device according to any of claims 1 to 12, **characterized in that** subunits composed of at least one semiconductor sensor are included as sensors (7.1) for the determination of gas concentrations.

14. A measuring device according to any of claims 1 to 13, **characterized in that** subunits composed according to the electrochemical principle are included as sensors (7.1) for the determination of gas concentrations.

15. A measuring device according to any of claims 1 to 14, **characterized in that** one additional subunit (7.n) is provided for measuring the particle concentration in gases by means of microbalance, light scattering or absorbance methods.

16. A measuring device according to any of claims 1 to 15, **characterized in that** additional sensors (7.n) for wind velocity, wind direction, air temperature, air pressure and air humidity, which are embedded in the measuring device, are provided.

17. A measuring device according to any of claims 1 to 16, **characterized in that** one additional subunit (7.n) is provided for the measurements of noise parameters such as, for example, acoustic pressure.

18. A measuring device according to any of claims 1 to 17, **characterized in that** sensors (7.n) for determining the contents of harmful substances in fluids, for example water, are provided as subunits which operate according to coloumetric methods.

19. A measuring device according to any of claims 1 to 18, **characterized in that** sensors (7.n) for determining the contents of harmful substances in fluids, for example water, are included as subunits which operate according to electrochemical methods, using in particular also ion-selective membranes.

20. A measuring device according to any of claims 1 to 19, **characterized in that** sensors (7.n) for determining the contents of harmful substances in fluids, for example water, are included as subunits which operate by determining the electrical conductivity.

21. A measuring device according to any of claims 1 to 20, **characterized in that** sensors (7.n) for determining the contents of harmful substances in fluids, for example water, are included as subunits which operate according to photometric methods.

22. A measuring device according to any of claims 1 to 21, **characterized in that** sensors (7.n) for determining the contents of harmful substances in fluids, for example water, are included as subunits which determine the TOC (Total Organic Compounds) content via methods of decomposing organic substances by conversion into carbon dioxide, applying, for example, a high-temperature treatment or using chemical reagents such as, for example, boric acid.

23. A measuring device according to any of claims 1 to 22, **characterized in that** at least one sensor (7.n) for determining the pH-value of fluids, for example water, is included.

24. A measuring device according to any of claims 1 to 23, **characterized in that** at least one sensor (7.n) for determining the BSB (biological oxygen demand) of fluids, for example water, is included.

25. A measuring device according to any of claims 1 to 24, **characterized in that** it is integrated into an advertising medium such as, for example, an advertising pillar or a large-scale display.

## Revendications

1. Système de mesure destiné à mesurer au moins un paramètre de l'environnement, comprenant un boîtier (1) fermé dans lequel sont agencés une alimentation centrale en courant (5), au moins un prélèvement d'échantillon (2) et une pompe (3) associée, au moins un capteur (7.1, 7.2, ...7.n) et une unité d'acquisition et de traitement de données (6) présentant une structure modulaire, **caractérisé en ce que** l'espace intérieur du boîtier (1), du point de vue de la régulation thermique, est divisé en un espace d'alimentation (1.2) uniquement aéré par une ventilation externe et un espace de mesure (1.1) comprenant un dispositif (4, 4.1) intégré permettant la climatisation de l'espace de mesure (1.1) et des capteurs (7.1, 7.2, ...7n) émettant une chaleur résiduelle ou leurs sous-composants, tels que par exemple des catalyseurs, sont protégés individuellement vis-à-vis de l'espace de mesure (1.1) par une isolation thermique (12.1, 12.2, ...12.n), la pompe (3) et l'alimentation en courant (5) étant agencées dans l'espace d'alimentation (1.2) et l'unité d'acquisition et de traitement de données (6) et le ou les capteurs (7.1, 7.2, ...) étant agencés dans l'espace de mesure (1.1).

2. Système de mesure selon la revendication 1, **caractérisé par** un dispositif (4) réalisé sous la forme d'un système de climatisation par compression servant à climatiser l'espace de mesure (1.1).

3. Système de mesure selon la revendication 1, **caractérisé par** un dispositif (4.1) servant à climatiser l'espace de mesure (1.1) comprenant au moins un élément Peltier à ventilation forcée.

4. Système de mesure selon l'une quelconque des revendications 1 à 3, **caractérisé par** des capteurs de température (9.1, 9.2, ...9.n) servant à surveiller la température des capteurs (7.1, 7.2, ...) ou de leurs sous-composants sous les isolations (12.1, 12.2, ...12.n).

5. Système de mesure selon l'une quelconque des revendications 1 à 4, **caractérisé en ce que** l'unité d'acquisition et de traitement des données (6), lors d'un dépassement continu de la température de fonctionnement admissible des capteurs (7.1, 7.2, ...7.n) ou des sous-composants sous les isolations (12.1, 12.2, ...12.n) individuelles, met ces derniers hors service.

6. Système de mesure selon l'une quelconque des revendications 1 à 5, **caractérisé en ce que** l'unité d'acquisition et de traitement de données (6) présente une structure modulaire comprenant au moins un processeur de signal (8.1, ...8.n) servant à capter et à convertir les signaux de mesure d'au moins un capteur (7.1, 7.2, ...7.n) en valeurs de tension et un processeur central (9) couplé au ou aux processeurs de signal (8.1...8.n) pour la conversion des valeurs de tension en valeurs de mesure de grandeurs physiques, le processeur central (9) étant couplé à une mémoire locale de valeurs de mesure (10).

7. Système de mesure selon la revendication 6, **caractérisé en ce que** le processeur central (9) est relié à une interface d'une unité de transfert de données (11).

8. Système de mesure selon la revendication 6 ou 7, **caractérisé en ce que** le processeur central (9) comprend une fonction de serveur web.

9. Système de mesure selon la revendication 3, **caractérisé en ce que** l'élément de Peltier est un élément de Peltier régulé monté sur une paroi extérieure du boîtier (1).

10. Système de mesure selon la revendication 1, **caractérisé par** un dispositif (4) réalisé sous la forme d'un mini-système de climatisation et servant à climatiser l'espace de mesure (1.1), lequel est intégré dans la paroi extérieure du boîtier (1).

11. Système de mesure selon l'une quelconque des revendications 1 à 10, **caractérisé en ce que** des sous-unités, lesquelles fonctionnent selon l'un des procédés de mesure physiques NDIR, NDUV, PID, FID, chimioluminescence ou absorption d'UV, sont utilisées comme capteurs (7.1) pour la détermination de concentrations de gaz.

12. Système de mesure selon l'une quelconque des revendications 1 à 11, **caractérisé en ce que** des sous-unités, lesquelles fonctionnent selon le principe électrochimique avec de l'oxyde de zircon ou selon le principe paramagnétique, sont utilisées comme capteur (7.1) de mesure de concentration d'oxygène dans des gaz.

13. Système de mesure selon l'une quelconque des revendications 1 à 12, **caractérisé en ce que** des sous-unités, lesquelles sont composées d'au moins un capteur à semiconducteur, sont utilisées comme capteurs (7.1) pour la détermination de concentrations de gaz.

14. Système de mesure selon l'une quelconque des revendications 1 à 13, **caractérisé en ce que** des sous-unités, lesquelles sont construites selon le principe électrochimique, sont utilisées comme capteurs (7.1) pour la détermination de concentrations de gaz.

15. Système de mesure selon l'une quelconque des revendications 1 à 14, **caractérisé en ce qu'**une sous-unité (7.n) servant à mesurer une concentration de particules dans des gaz au moyen d'une microbalance, d'un procédé de diffusion ou d'extinction de lumière, est prévue en plus.

16. Système de mesure selon l'une quelconque des revendications 1 à 15, **caractérisé en ce que** des capteurs (7.n) de la vitesse du vent, du sens du vent, de la température de l'air, de la pression de l'air et de l'humidité de l'air, lesquels sont intégrés dans le système de mesure, sont prévus en plus.

17. Système de mesure selon l'une quelconque des revendications 1 à 16, **caractérisé en ce qu'**une sous-unité (7.n) pour les mesures de paramètres de bruits, tels que par exemple la pression acoustique, est prévue en plus.

18. Système de mesure selon l'une quelconque des revendications 1 à 17, **caractérisé en ce que** des capteurs (7.n) servant à déterminer les teneurs en polluants dans des liquides, par exemple de l'eau, sont prévus comme sous-unités, lesquelles fonctionnent selon le procédé colorimétrique.

19. Système de mesure selon l'une quelconque des revendications 1 à 18, **caractérisé en ce que** des capteurs (7.n) servant à déterminer des teneurs en polluants dans des liquides, par exemple de l'eau, sont utilisés comme sous-unités, lesquelles fonctionnent selon le procédé électrochimique, en particulier également au moyen de membranes sélectives d'ions.

20. Système de mesure selon l'une quelconque des revendications 1 à 19, **caractérisé en ce que** des capteurs (7.n) servant à déterminer des teneurs en polluants dans des liquides, par exemple de l'eau, sont utilisés comme sous-unités, lesquelles fonctionnent au moyen de la détermination de la conductivité électrique.

21. Système de mesure selon l'une quelconque des revendications 1 à 20, **caractérisé en ce que** des capteurs (7.n) servant à déterminer des teneurs en polluants dans des liquides, par exemple de l'eau, sont utilisés comme sous-unités, lesquelles fonctionnent selon le procédé photométrique.

22. Système de mesure selon l'une quelconque des revendications 1 à 21, **caractérisé en ce que** des capteurs (7.n) servant à déterminer des teneurs en polluants dans des liquides, par exemple de l'eau, sont utilisés comme sous-unités, lesquelles déterminent la teneur en TOC (composants organiques totaux) par l'intermédiaire du procédé de la désagrégation des substances organiques par conversion en dioxyde de carbone, par exemple par traitement à haute température ou au moyen de réactifs chimiques, tels que par exemple l'acide de bore.

23. Système de mesure selon l'une quelconque des revendications 1 à 22, **caractérisé en ce qu'**au moins un capteur (7.n) servant à déterminer le pH de liquides, par exemple de l'eau, est utilisé.

24. Système de mesure selon l'une quelconque des revendications 1 à 23, **caractérisé en ce qu'**au moins un capteur (7.n) servant à déterminer les BSB (besoins biologiques en oxygène) de liquides, par exemple de l'eau, est utilisé.

25. Système de mesure selon l'une quelconque des revendications 1 à 24, **caractérisé en ce qu'**il est intégré dans un support publicitaire, tel que par exemple dans une borne publicitaire ou un affichage de grande taille.
